# EUROPEAN PATENT SPECIFICATION

(11) **EP 1 591 124 B1**
(45) Date of publication and mention of the grant of the patent: **12.09.2007**
(21) Application number: 05425064.2
(22) Date of filing: 10.02.2005
(51) Int. Cl.: A61K 35/18, A61P 9/14

(54) **Galenic preparation for anal administration in the treatment of haemorrhoids**
Arzneimittel zur analen Behandlung von Hämorrhoiden
Forme galenique pour l'administration anal dans le traitement thérapeutique d'hémorroides

(30) Priority: 28.04.2004 IT dp20040003
(43) Date of publication of application: 02.11.2005
(73) Proprietor: Sciaraffa Ciro, 84134 Salerno (IT)
(72) Inventor: Sciaraffa Ciro, 84134 Salerno (IT)

(56) References cited:
- WO-A-94/25041
- ANONYMOUS: "Natural healing with horsetail"[Online] 25 February 2004 (2004-02-25), pages 1-10, XP002332645 Retrieved from the Internet: URL:http://www.lifestyle-weblog.com/502267 11/february_2004.php> [retrieved on 2005-06-20]
- ANONYMOUS: "Horsetail (Equisetum arvense)"[Online] 1999, - 2004 pages 1-4, XP002332646 Retrieved from the Internet: URL:http://www.rain-tree.com/horsetail.htm > [retrieved on 2005-06-20]
- DATABASE WPI Section Ch, Week 200536 Derwent Publications Ltd., London, GB; Class B04, AN 2005-347216 XP002332647 & BR 200 301 056 A (MACHADO DA CRUZ J R) 10 May 2005 (2005-05-10)

## Description

The galenic preparation of the present application is a product obtained from a plant called Equisetum arvense, also known as "Coda Cavallina"; subjected to a simple and systematic process in water this plant yields a fluid indicated in the treatment of internal and external haemorrhoids, when introduced by enteroclysis or applied locally by means of a soaked pledget, respectively, with consequent improvement of the complexion of the individual.

The galenic preparation permits the treatment of haemorrhoids whit a natural product of vegetable origin, in liquid state, than can be administered through enteroclysis or applied locally by means of pledgets soaked in the fluid, therefore avoiding the traditional use of ointments or suppositories or even the troublesome surgical treatment, while assuring definite and satisfying results and also, considering the low costs of productions, significant economic advantages.

It is known from prior art documents that E. arvense has therapeutical properties and is used for haemorrhoidal tissue repair, see for example http://www.lifestyle-weblog.com/502267 11/february_2004.php. However, in this document is not mentioned the product obtainable from these plant, nor is there any description of the preparation procedure, dosage of method of administration.

E. arvense is known to have antihaemorrhagic properties, and is useful for the rapid healing of wounds, see http://www.rain-tree.com/horsetail.htm, but this document says nothing about the type of preparation obtainable from Equisetum and that can be used directly, nor does it describe dosages or duration of treatment.

WO 94/25041 reveals the properties of Equisetum for the treatment of skin associated pathologies, based on the use of a juice extracted from the plant, diluted in ethanol or other liquid vehicle, but there is no reference to haemorrhoids.

WO 97/25997 speaks of an antihaemorrhoidal product that can be obtained from brewing a mixture of herbs belonging to the species Equisetum and Julianiceae, to be administered per os.

Finally, in RU2133618 (database DERWENT abstract 2000-363139 [31]) is described an infusion obtained from a mixture of Equisetum an other different plants, administered per os for the treatment of haemorrhoids.

### A - The preparation can be obtained from Equisetum arvense and obtained with the procedure divided in the following steps:

Phase I. - Gathering the plants. The picking of the plants is best between July and September, when they are verdant and lush. Even though the entire plant can be used efficaciously, even if picked in autumn, to get the most from the vegetable it is advisable to sever the plant at 5 or 6 cm from the ground.
Phase II. Cleaning the plants. After the picking, the plants have to thoroughly cleaned and the weeds eventually present removed.
Phase III. Cutting the plants. Cleaned the plants, these are divided by severing them between each node, while the tops are cut in pieces of 4 to 5 centimetres.
Phase IV. Washing. The plants thus cut and chopped are thoroughly and repeatedly washed in warm water, till all impurities are washed away, as demonstrated by the draining of clear water.
Phase V. Preparing the Boiling of the vegetable. The clean vegetables obtained in the previous phase are placed in a enamelled o non enamelled steel pot or in an aluminium pot, with enough water to reach a level of approximately 1 cm above the mass of packed vegetables. Otherwise, a useful guide is to add 1 litre of water for every 180 to 200 g of fresh vegetables.
Phase VI. Boiling the vegetables in order to obtain the liquid preparation. To obtain a product useful for the treatment of internal haemorrhoids, the plants should be boiled from 40 to 80 minutes; on the other hand, protracting boiling from 80 to 160 minutes allows to obtain a liquid preparation efficacious for the treatment of external haemorrhoids.
Phase VII. Draining and Bottling. After boiling, the product is left standing still for about 3 to 4 hours and than filtered across two layers of a tight filtering material and subsequently poured in accurately labelled, well closed glass containers of 750 ml, or in an apposite plastic enema of approximately 150 ml.

### B - Method of administration:

Part I. Method of administration for the treatment of internal haemorrhoids. The full treatment of internal haemorrhoids consists of 5 (five) enteroclysis of approximately 150 ml each, one every night for 5 consecutive nights, preferably after bowel movement.
-Part II. Method of administration for the treatment of external haemorrhoids with partial atrophy of varix. A certain improvement of external haemorrhoids can be obtained after local application by means of cotton ball pledgets wrapped in sterile gauze and soaked with 2-3 teaspoons or 6-9 ml of the liquid preparation obtained after the second boiling, or even that obtained after the first boiling for the less severe cases. The pledget is applied in direct contact with the protruding haemorrhoids and left till dry, 3 to 4 timed a day, from 3 to 5 consecutive days.

### C - Effects of the preparation:

Part I. Effects of the treatment on internal haemorrhoids. After the administration of 5 enteroclysis (but a certain improvement should already be evident after the third administration), the patient should experience arrest of blood loss (if present) and relief of pain, with significant increase in comfort for the individual; furthermore, this type of treatment is free of undesired effects and assures, especially when associated with a correct diet, a complete cure in a period of time that however cannot be determined beforehand, and contemporarily the patient experiences an amelioration of his aspect and complexion.
Part II. Effects of the treatment of external haemorrhoids. After the application of the soaked pledgets as indicated in Point B, Part II, (three-four times a day for three to five consecutive days), the patient will experience a certain improvement of his condition and in a short while relief of pain, as a consequence of the partial atrophy of haemorrhoids.

Practically, details regarding the gathering of the plant, preparation of the product, dosages and methods of administration may have slight variations, that however going do not go beyond the domain of the application.

## Claims

1. A galenic liquid preparation obtainable by decoction from Equisetum arvense plants for use as a medicament for treating internal or external haemorrhoids, by rectal administration of 5 enteroclysis of approximately 150 ml each, one every night for 5 consecutive nights, or by application of pledgets wrapped in sterile gauze soaked with two or three teaspons or 6-9 ml of the liquid, respectively.

2. A method for preparing the natural galenic preparation of claim 1 by boiling E. arvense plants from 40 to 160 minutes.

3. The method of claim 2 wherein the boiling of E. arvense plants is performed from 40 to 80 minutes.

4. The method of claim 2 wherein the boiling of E. arvense plants is performed from 80 to 160 minutes.

5. A liquid preparation for use as a medicament for the treatment of haemorrhoids obtainable from E. arvense according to the following procedure:
a) the plant is prepared by cutting it along the stalk, between the nodes for a major yield;
b) cleaning and washing in warm water;
c) boiling: the cut and cleaned plants are boiled in a steel pot or other appropriated container filed with enough water to reach a level of approximately 1 cm above the mass of packed vegetables or 1 liter of water for every 180 to 200g of the fresh raw plant;
d) boiling the plant from 40 to 80 minutes, in order to obtain a preparation appropriate for the treatment of internal or external haemorrhoids, or from 80 to 160 minutes for a preparation indicated for the treatment of external haemorrhoids.

6. The use of the galenic preparation of claim 1 for the manufacture of a medicament for:
a) the treatment of internal haemorrhoids wherein the dose is 150 ml of the liquid preparation administered by enteroclysis for five consecutive nights, preferably after bowel movement;
b) the treatment of external haemorrhoids wherein cotton ball pledgets wrapped in sterile gauze soaked with two or three teaspoons or 6-9 ml of the galenic preparation obtained by the method of claim 4 are applied locally 3-4 times a day, from 3 to 5 consecutive days, and wherein the pledget is left in place till it dries

## Patentansprüche

1. Eine galenische Flüssigkeit, die durch Absieden aus der Pflanze Equisetum arvense gewonnen wird, und die als Medikament zur Behandlung von inneren und äußeren Hämorrhoiden anzuwenden ist. Im ersten Fall durch rektale Verabreichung von 5 Klistierspritzen à 150 ml abends während fünf aufeinander folgender Tage, im zweiten Fall mittels Auftragen eines sterilen Gazebausches, der mit zwei bis drei Löffelchen der Zubereitung (ca. 6-9 ml) getränkt wurde.

2. Eine Methode der Zubereitung des in Punkt 1) beschriebenen natürlichen galenischen Produkts, die auf dem Absieden der Pflanze E. arvense für 40-160 Min. beruht.

3. Die in Punkt 2) beschriebene Methode, wobei sich die Kochzeit der Pflanze E. arvense von 40 auf 80 Min. verlängert.

4. Die in Punkt 2) beschriebene Methode, wobei sich die Kochzeit der Pflanze E. arvense von 80 auf 160 Min. verlängert.

5. Eine flüssige Zubereitung, die als Medikament zur Behandlung der Hämorrhoiden zu verwenden ist, und die aus der Pflanze E. arvense durch das folgende Verfahren gewonnen wird:
a) die Pflanze wird längs des Stängels zwischen den Knotenstellen aufgeschlitzt, um eine bessere Ausnutzung zu erreichen;
b) Säubern und Waschen in lauwarmem Wasser;
c) Sieden: die so gesäuberten und zerkleinerten Pflanzen werden in einem Stahl- oder anderen geeigneten Topf gekocht. Dabei muss das Wasser ca. 1 cm über den satt eingefüllten Pflanzen stehen; das Verhältnis ist 1 Liter Wasser pro 180-200 gr. Frischpflanze. Zubereitung für die Behandlung der inneren Hämorrhoiden, oder 80-160 Minuten, um die Zubereitung für die Behandlung der äußeren Hämorrhoiden zu gewinnen.

6. Die Verwendung des in Punkt 1) beschriebenen galenischen Produkts zur Herstellung eines Medikaments, das geeignet ist für:
a) die Behandlung der inneren Hämorrhoiden durch die Verabreichung von 150 ml-Klistierspritzen der flüssigen Zubereitung für fünf aufeinander folgende Abende, vorzugsweise nach der Darmentleerung;
b) die Behandlung äußerer Hämorrhoiden durch 3-4-maliges lokales Auftragen für 3-5 aufeinander folgende Tage mittels eines in sterile Gaze gewickelten Wattebausches, der mit zwei bis drei Löffelchen (6-9 ml) der durch die in Punkt 4) beschriebenen Methode gewonnenen galenischen Zubereitung getränkt wurde, und der bis zur Austrocknung an Ort gelassen wird.

## Revendications

1. La préparation du liquide galénique est obtenue par décoction de la plante Equisetum arvense afin d'être utilisée en tant que médicament pour le soin des hémorroïdes internes ou externes. L'application de ce liquide s'effectue soit par voie rectale avec 5 entéroclysmes d'environ 150 ml chacun (un par soir, pendant cinq jours consécutifs), soit par application d'un tampon de gaze stérilisée, imbibé de deux ou trois cuillères de ce liquide, correspondant à environ 6-9 ml.

2. La méthodologie de préparation de ce produit galénique naturel est décrite dans la revendication 1, qui est basée sur une ébullition de la plante E. arvense pendant 40-160 minutes.

3. La méthodologie mentionnée dans la revendication 2 est basée sur une ébullition de la plante E. arvense pouvant durer de 40 à 80 minutes.

4. La méthodologie mentionnée dans la revendication 2 est basée sur une ébullition de la plante E. arvense pouvant durer de 80 à 160 minutes.

5. La préparation de ce liquide, en tant que médicament pour le traitement des hémorroïdes, est obtenu grâce à la plante E. arvense selon le processus suivant :
a) La plante est incisée le long de sa tige, entre les différents noeuds, de sorte à augmenter son efficacité ;
b) Nettoyage et lavage en eau tiède;
c) Ébullition : les plantes, ainsi nettoyées et déchiquetées, sont bouillies dans une casserole en acier ou dans un autre récipient approprié, rempli d'eau en quantité suffisante pour couvrir les plantes jusqu'à une hauteur d'environ un centimètre, ou alors en respectant les proportions de 180 à 200 grammes de plante fraîche par litre d'eau.
d) Ébullition de la plante pendant 40-80 minutes afin d'obtenir la préparation appropriée au traitement des hémorroïdes internes ou externes, ou alors une ébullition de 80-160 minutes pour la préparation appropriée au traitement des hémorroïdes externes.

6. L'utilisation de ce produit galénique est indiquée dans la revendication 1 en tant que médicament pour les usages suivants :
a) Le traitement des hémorroïdes internes s'effectue par application d'entéroclysmes de 150 ml pendant 5 soirs consécutifs, de préférence après la défécation ;
b) Le traitement des hémorroïdes externes s'effectue par application locale, 3-4 fois par jour pendant 3-5 jours consécutifs, d'un tampon d'ouate recouvert de gaze stérilisée et imbibé de deux à trois cuillères du liquide galénique (soit 6-9 ml) obtenu selon la méthodologie indiquée dans la revendication 4 ; ce tampon devra rester sur la partie intéressée jusqu'à ce qu'il ne soit bien sec.
